# EUROPEAN PATENT SPECIFICATION

(11) **EP 1 289 569 B1**
(45) Date of publication and mention of the grant of the patent: **16.08.2006**
(21) Application number: 00931515.1
(22) Date of filing: 09.06.2000
(51) Int. Cl.: A61K 49/00

(54) **DIAGNOSING COLIC TYPE IN AN INFANT**
DIAGNOSE EINES KOLIK-TYP IN DER KINDERHEILKUNDE
DIAGNOSTIQUER LE TYPE DE COLIQUE CHEZ LE NOURRISSON

(43) Date of publication of application: 12.03.2003
(73) Proprietor: Crosscare Research & Development Limited, Dublin 24 (IE)
(72) Inventor: BUCKLEY, Mary, Margaret, Cork, County Cork (IE); CLAYTON, Paul, Rodney, Bletchingley, Surrey RH1 4QZ (GB); TIERNEY, Jonathan, James, Ranelagh, Dublin 6 (IE)
(74) Representative: Ryan, Anne Mary
(86) International application number: PCT/IE2000/000075
(87) International publication number: WO 2001/093915

(56) References cited:
- LIN, MEEI YN ET AL: "Comparative effects of exogenous lactase (.beta.-galactosidase) preparations on in vivo lactose digestion" DIG. DIS. SCI. (1993), 38(11), 2022-7 , XP000990329
- DAVID GAON ET AL.: "Digestion de la Lactosa por una leche fermentada con lactobacillus acidophilus y lactobacillus casei de origin humano" MEDICINA, vol. 55, 1995, XP000990326 Buenos Aires
- DATABASE EMBASE [Online] ELSEVIER SCIENCE PUBLISHERS, AMSTERDAM, NL; BARR R.G. ET AL: "Breath hydrogen excretion in normal newborn infants in response to usual feeding patterns: Evidence for 'functional lactase insufficiency' beyond the first month of life." retrieved from STN Database accession no. 84100623 XP002163173 & JOURNAL OF PEDIATRICS, (1984) 104/4 (527-533). CODEN: JOPDAB,
- DATABASE EMBASE [Online] ELSEVIER SCIENCE PUBLISHERS, AMSTERDAM, NL; MACLEAN JR. W.C. ET AL: "Lactose malabsorption by premature infants: Magnitude and clinical significance." retrieved from STN Database accession no. 81002054 XP002163174 & JOURNAL OF PEDIATRICS, (1980) 97/3 (383-388). CODEN: JOPDAB,
- J.J. MILLER ET AL.: "Effect of Lactase on infantile colic" JOURNAL OF PEDIATRICS, (1984) 104/4 (993-994). CODEN: JOPDAB, XP000990328
- DATABASE MEDLINE [Online] US NATIONAL LIBRARY OF MEDICINE (NLM), BETHESDA, MD, US; LADAS S ET AL: "Lactose malabsorption in Greek adults: correlation of small bowel transit time with the severity of lactose intolerance." retrieved from STN Database accession no. 83028713 XP002163175 & GUT, (1982 NOV) 23 (11) 968-73. ,

## Description

### Technical Field.

This invention relates to a method of distinguishing between different types of colic in infants and as a result allowing for more effective management and therapy of colic in such infants.

### Background Art

Infant colic affects between 10% and 30% of babies between 3 and 13 weeks (Weisbult, H. (1994) Current Pediatric Therapy (14^{th} ed) Philadelphia Saunders, pp 233-236), whether breast-or formula-fed. Although it is not generally considered to be a serious medical condition, colic can lead to failure to thrive and in extreme cases to dehydration and electrolyte imbalance. More commonly it may contribute to impaired parent-child bonding, and increase the risk of child abuse (Wolke, D. *et al* (1994) Pediatr. *94*: 322-332). The aetiology of the condition is unclear, and this has led to a proliferation of treatment strategies, none of which is very successful. Indeed, one of the most commonly used remedies (dimethicone) was shown to be no better than placebo in two double-blind cross-over studies (Metcalfe T.J. *et al* (1994) Paediatr. *94*: 29-34).

One current theory suggests that colic may be caused by transient relative lactase deficiency, which may reflect an immature digestive system (Barr, R.G. *et al* (1984) J. Pediatr. *104:* 527-533). The resulting failure to break down all the lactose in the feed allows significant amounts of lactose in breastmilk or formula to enter the large bowel. It then becomes a substrate for lactobacilli and bifidobacteria in the colon, which break it down in a fermentation reaction to produce various acids, including acetic, propionic and butyric acids and hydrogen. The subsequent increase in breath hydrogen is an accepted indirect biomarker for hypolactasia (Levitt '69), and has been reported in infants with colic (Moore D.J. *et al* (1988) J. Pediatr, Vol. 113, p979-984) The rapid production of hydrogen in the lower bowel distends the colon, causing pain. The osmotic pressures generated by the lactose and lactic acid in the colon cause an influx of water, leading to further distension and acidic diarrhoea.

This model of colic implies that symptoms could be relieved by reducing the lactose content of the infant's feed. This hypothesis has been tested in a small double-blind study in which the feed of colicy babies was pre-incubated with lactase (Kearney P.J. *et al* (1998) J Hum Nut. Dietetics, *11:* 281-285). The results were positive, but the trial size (n=13) precluded formal proof. Kearney *et al* found that all thirteen infants were responders which would appear to be statistically anomalous as in a representative population all of the infants presenting with symptoms of colic would not all be expected to be suffering from transient lactose intolerance.

Accordingly, there is a need for a method of diagnosing colic type in infants, especially as failure to identify colic type at an early date can have serious consequences. In infants whose colic is caused by factors other than transient lactose intolerance, such as carry food allergies, low-level infection or metabolic errors, failure to diagnose such conditions can have serious and often irreversible effects.

### Disclosure of Invention

The invention provides use of a β-D-galactosidase enzyme for the manufacture of a diagnostic agent for diagnosing colic type in an infant exhibiting one or more symptoms characteristic of unspecified colic, which comprises the steps of:
a) optionally measuring in said infant at least one parameter indicative of unspecified colic;
a) orally administering a liquid formulation of said β-D-galactosidase enzyme separately, simultaneously with or sequentially to a lactose-containing feed; and
b) determining at selected intervals of time any alteration in said infant of colic-type symptoms.

In one aspect of the invention, an improvement in said symptoms of at least 50% or greater is indicative of lactose-based intolerance.

Accordingly, where administration of lactase results in a positive result, the infant should remain on lactase therapy for a period of three months or until symptoms no longer present or as the attending clinician may prescribe.

In an alternative aspect of the invention, when said enzyme formulation fails to alleviate the symptoms of unspecified colic, the infant is then placed on a diet of low allergen feed.

In a further aspect of the invention, an improvement in said symptoms of 50% or more is indicative of allergen-based colic requiring maintenance on a low allergen-based diet.

If food allergy, principally bovine serum albumin (BSA) allergy, is the cause of the colic, then the symptoms will be alleviated by a low allergen feed.

Furthermore, if early BSA allergy is a significant cause of infant colic, as has been suggested, non-responders to lactase may include significant numbers of early atopy. As BSA allergy is increasingly regarded as the cause of Type 1 diabetes (Karjalainen, J. *et al* (1992) New Eng. J. Med. 327: 302-307) the use of lactase in accordance with the invention will help to identify those infants where BSA allergy is developing, and enable the clinician to initiate dietary shifts (dairy to soy) which could reduce the numbers of Type 1 diabetics. This would in turn lead to considerably reduced healthcare costs for this life-long disease.

Accordingly, it will be appreciated that application of the invention would result in additional, longer-term health and medeconomic benefit.

The benefits to both infants and their parents will be appreciated, because in the majority of infants presenting with unspecified colic, effective management and therapy can be implemented at a very early stage, namely at the onset of symptoms. The benefits to healthcare visitors and other healthcare professionals, including A & E departments and paediatricians, will result in fewer calls by concerned parents with the attendant advantages.

In a still further aspect of the invention a failure to respond is indicative of a need for further investigation.

In one embodiment of the invention when said infant is fed a man-made formula, said formula is pre-incubated with said enzyme.

In an alternative embodiment of the invention, wherein when said infant is breast-fed, said enzyme is added to an aliquot of fore-milk and then administered to the infant at the end of a feed.

Preferably, the parameter measured is selected from one or more of the following: breath hydrogen, stool pH, stool reducing substances; and bowel distension.

In a preferred embodiment of the invention there is provided use of a β-D-galactosidase enzyme for the manufacture of a diagnostic agent for diagnosing colic type in an infant exhibiting one or more symptoms characteristic of unspecified colic, which comprises the steps of:
a) measuring breath hydrogen in said infant;
b) orally administering a liquid formulation of said β-D-galactosidase enzyme separately, simultaneously with or sequentially to a lactose-containing feed;
c) measuring breath hydrogen at intervals over a period of at least one hour; and
d) determining the difference in breath hydrogen relative to said pre-feed measurement.

In one embodiment of this aspect of the invention a reduction in said breath hydrogen of the order of 50% or more relative to said pre-feed measurement is indicative of lactose intolerance-based colic.

In an alternative embodiment of this aspect of the invention a failure to respond to said enzyme formulation as measured by a breath hydrogen which is substantially unchanged relative to said pre-feed measurement is indicative of colic other than lactose intolerance-based colic.

Preferably, the breath hydrogen is measured at time intervals of 30, 60, 190 and 120 min. post lactose-based feed.

Further preferably, the breath hydrogen is measured by means of a portable hydrogen monitor.

A suitable probable hydrogen monitor is a Micro H₂ (Micro Medical Ltd, Chatman, UK). This is a portable hydrogen monitor designed for the simple screening of lactose or sucrose malabsorption by automatic sensor drift detection (Peuhkuri K. *et al* (1998) Scand. J. Clin. Invest. *58*: 217-224).

Preferably, the enzyme is a β-galactosidase obtained from the controlled fermentation of *Kluyveromyces lactis.*

A β-galactosidase (lactase) preparation is commercially available as a in over-the-counter preparation sold under the trade mark COLIEF. (COLIEF is a registered trade mark of Crosscare Limited, Dublin, Ireland).

Further, preferably, the diagnosis is confirmed by an alteration in cry time.

The invention will be further illustrated by the following Example.

### Mode for Carrying Out the Invention

### Example

### Double-blind randomised entry study with cross-over to investigate colic type in infants exhibiting one or more symptoms of unspecified colic.

A double-blind study with randomised entry and cross-over was set up to investigate colic type in infants exhibiting one or more symptoms of unspecified colic. Infants who met the trial criteria were assessed in both limbs of the study for colic symptoms and breath hydrogen as hereinbelow described.

### Experimental Procedures

### Subjects

Fifty three infants aged between 3 and 13 weeks were recruited. New mothers were given trial-related literature in the recovery rooms, and contacted by phone in the following 3 to 4 weeks to see if their newborn was presenting symptoms of colic. Follow-up was by a midwife who visited the parents in their homes, and determined acceptability according to the trial criteria. These included cry time *per* 24 hours, incidence of spasm, and diarrhoea, and are in line with Wessel's criteria (Wessel M.A. *et al* (1954) Pediatr. *14*: 421-424), except that a 3 week duration of symptoms was not required. Parental consent was obtained after full explanation of the purpose and nature of the trial, and the study was approved by the Guy's & St Thomas Ethics Committee.

### Materials

The β-galactosidase (lactase) used was COLIEF (registered trade mark) as described above. COLIEF and a heat-inactivated placebo were obtained from the manufacturers.

### Breath hydrogen measurement

Breath hydrogen was measured using a Micro H₂ as hereinabove described. Samples were taken using face-masks with one-way valves, before feeding, and at 10 minute intervals thereafter, up to a maximum of 120 minutes.

### Experimental design

The trial design was double-blind. Subjects were randomly assigned to the verum or placebo arm for an initial period of ten days, and then switched after a 5 day wash-out phase to ten days in the alternate arm. The severity of symptoms of colic was assessed by the parents, who filled in a daily record denoting cry time, incidence of spasm, stool habit and feed details. Breath hydrogen testing was performed by a health visitor where possible during the last two days at the end of each arm.

The preparations were given in bottles marked with the infant's entry code, and either 'A' or 'B', to maintain the double-blind. All bottles were retrieved at the end of the two courses in order to ascertain compliance.

### Lactase incubation

In formula-fed babies, mothers were instructed to add 3 drops of COLIEF or placebo to each (cooled or body temperature) feed, shake gently, refrigerate for approximately 4 hours before re-warming and giving as normal. A similar procedure has been shown to be effective in reducing milk osmolality in vitro (Malone, A.J. *et al* (1995) Irish J. Med. Sci. *164:* 22).

In breast-fed babies, mothers were instructed to express the fore-milk (which has a particularly high lactose content) into a tea-spoon, and add to this 3 drops of COLIEF. They then breast-fed as usual, and gave the treated fore-milk to the infant at the end of the feed. The relatively short incubation period in this case is compensated for by the higher concentration of lactase in the small volume of expressed fore-milk.

### Results

Fifty three subjects entered the trial as indicated above. There were 6 drop-outs, 1 subject mislaid the case record form (CRF), and 1 subject who appeared on checking to be inelligible; leaving 45 subjects who were processed through the trial.

Of these 45 subjects, 4 were judged to be non-compliant based on the bottle returns (i.e. the bottle was not used), and showed no reduction in crying time. In this group, breath hydrogen measurements were not carried out. It was considered that nothing useful was known about this group, and thus they were discarded from further consideration. This left 41 subjects who were considered to have validity. This is the figure used in subsequent calculations on % responders and non-responders.

Of the 41 valid subjects, an additional 5 subjects were non-compliant, but when challenged with a COLIEF dose for breath testing, showed a nul result. This group were considered to be non-responders.

Of the remaining 36 subjects who were compliant, another 11 were non-responders.

Summing groups C and D gave a total of 16/41 non-responders, and left a residual number of 25/41 subjects who can be described as compliant, and variously responsive to COLIEF treatment.

Of the 25 subjects, 8 reduced cry time and breath hydrogen by 50% or more.

An additional 2 subjects reduced cry time and breath hydrogen by within 5% of the 50% margin. Because cry time and breath hydrogen shifted significantly and in parallel, these subjects were considered to be responders.

A further 2 subjects showed a 50% reduction in cry time, but were not breath tested.

A further 7 subjects showed a 50% reduction in cry time, but did not show any significant breath hydrogen shifts.

In responders on placebo, baseline scores were: 5.2 ppm, rising at 30, 60, 90 and 120 min. to 5.7, 7.3, 9, 7.5, 7.8 ppm. On verum, their baseline score was 1.8 ppm, and at 30, 60, 90 and 120 min. this shifted to 1.8, 1.5, 1.5, 1.5 ppm respectively.

The above figures represent average readings, taken from the set of responders. It will be observed that the differences between verum and placebo are significant.

In the subjects who showed a 50% reduction in cry time but did not show any significant breath hydrogen shifts, this may be due to different bacterial strains colonising the gut in those subjects. Different bacterial strains produce different amounts of gas as a result of fermentation. In such subjects it is possible that the colic symptoms are mediated by the acid produced.

The final 4 subjects were non-compliant, as measured by bottle returns, and unsurprisingly showed no change in cry time. However, when challenged with verum, they showed a 3-fold or more reduction in breath hydrogen. This could be considered to be a positive response. However, it was decided to discard this group on the basis that they were inadequately characterised.

If Group 1 are defined as responders, then it can be said that of the 41 subjects who were tested, 16 were non-responders (39%), and 25 were responders (61%). More conservatively, if Group I is discarded, the data reconfigures as 16/37 non-responders (43%), and 21/37 responders (57%).

In the above study, only half of the subjects responded to lactase, but in the group that did respond the treatment was very effective, reducing cry time and/or breath hydrogen by half or more. The above results can be interpreted as follows.

The diagnostic entity of infant colic does not describe aetiology, but is merely a description of symptoms. In these situations, it is not unusual to find that new analytical techniques may reveal that the condition is heterogeneous, with different underlying pathophysiology and aetiology. The above trial can be interpreted as showing that the difference between responders and non-responders is due to different aetiologies.

## Claims

1. Use of a β-D-galactosidase enzyme for the manufacture of a diagnostic agent for diagnosing colic type in an infant exhibiting one or more symptoms characteristic of unspecified colic, which comprises the steps of:
a) optionally measuring in said infant at least one parameter indicative of unspecified colic;
b) orally administering a liquid formulation of said β-D-galactosidase enzyme separately, simultaneously with or sequentially to a lactose-containing feed; and
c) determining at selected intervals of time any alteration in said infant of colic-type symptoms.

2. Use according to Claim 1, wherein an improvement in said symptoms of at least 50% or greater is indicative of lactose-based intolerance.

3. Use according to Claim 1, wherein when said enzyme formulation fails to alleviate the symptoms of unspecified colic, the infant is then placed on a diet of low allergen feed.

4. Use according to Claim 3, wherein an improvement in said symptoms of 50% or more is indicative of allergen-based colic requiring maintenance on a low allergen-based diet.

5. Use according to Claim 3, wherein a failure to respond is indicative of a need for further investigation.

6. Use according to any preceding claim, wherein when said infant is fed a man-made formula, said formula is pre-incubated with said enzyme.

7. Use according to any one of Claims 1-5, wherein when said infant is breast-fed, said enzyme is added to an aliquot of fore-milk and then administered to the infant at the end of a feed.

8. Use according to any preceding claim, wherein the parameter measured is selected from one or more of the following: breath hydrogen, stool pH, stool reducing substances; and bowel distension.

9. Use of a β-D-galactosidase enzyme for the manufacture of a diagnostic agent for diagnosing colic type in an infant exhibiting one or more symptoms characteristic of unspecified colic, which comprises the steps of:
a) measuring breath hydrogen in said infant;
b) orally administering a liquid formulation of said β-D-galactosidase enzyme separately, simultaneously with or sequentially to a lactose-containing feed;
c) measuring breath hydrogen at intervals over a period of at least one hour; and
d) determining the difference in breath hydrogen relative to said pre-feed measurement.

10. Use according to Claim 9, wherein a reduction in said breath hydrogen of the order of 50% or more relative to said pre-feed measurement is indicative of lactose intolerance-based colic.

11. Use according to Claim 9, wherein a failure to respond to said enzyme formulation as measured by a breath hydrogen which is substantially unchanged relative to said pre-feed measurement is indicative of colic other than lactose intolerance-based colic.

12. Use according to any one of Claims 9-11, wherein the breath hydrogen is measured at time intervals of 30, 60, 90 and 120 min. post lactose-based feed.

13. Use according to any preceding claim, wherein the breath hydrogen is measured by means of a portable hydrogen monitor.

14. Use according to any preceding claim, wherein the enzyme is a lactase obtained from the controlled fermentation of *Kluyveromyces lactis.*

15. Use according to any preceding claim, wherein the diagnosis is confirmed by an alteration in cry time.

## Patentansprüche

1. Verwendung eines β-D-Galaktosidase-Enzyms für die Herstellung eines diagnostischen Wirkstoffes für die Diagnose einer Kolikart, die in einem Kind ein oder mehrere Symptome zeigt, die charakteristisch für eine unspezifische Kolik sind, wobei die Diagnose folgende Schritte umfasst:
a) optionale Messung mindestens eines Parameters, der unspezifische Kolik in dem Kind anzeigt;
b) orale Verabreichung einer flüssigen Formulierung des β-D-Galaktosidase-Enzyms separat von, simultan mit oder auf eine Laktose-enthaltende Nahrung folgend; und
c) Bestimmung jeder Veränderung in dem Kind, hinsichtlich der Symptome der KolikArt nach ausgewählten Zeitintervallen.

2. Verwendung nach Anspruch 1, wobei eine Verbesserung der Symptome von mindestens 50 % oder mehr eine Laktose-basierende Intoleranz anzeigt.

3. Verwendung nach Anspruch 1, wobei, wenn die Enzymformulierung bei der Linderung der Symptome der unspezifischen Kolik fehlschlägt, das Kind dann auf Diät mit schwachallergener Nahrung gesetzt wird.

4. Verwendung nach Anspruch 3, wobei eine Verbesserung der Symptome um 50 % oder mehr eine allergen-basierende Kolik anzeigt, die der Versorgung einer schwach Allergenbasierenden Diät bedarf.

5. Verwendung nach Anspruch 3, wobei ein Fehlschlag der Reaktion indikativ für die Notwendigkeit einer weiteren Untersuchung ist.

6. Verwendung nach einem der vorhergehenden Ansprüche, wobei, wenn das Kind mit einer von Menschen hergestellten Formulierung gefüttert wurde, die Formulierung mit dem Enzym vorinkubiert wurde.

7. Verwendung nach einem der Ansprüche 1 bis 5, wobei, wenn das Kind ein Brustkind ist, das Enzym zu einem Aliquot der Vormilch zugegeben wird, und dann am Ende der Fütterung dem Kind verabreicht wird.

8. Verwendung nach einem der vorhergehenden Ansprüche, wobei der gemessene Parameter aus einem oder mehreren der Folgenden ausgewählt ist: Wasserstoffgehalt der Atemluft, pH-Wert des Kots, Kot-reduzierende Substanzen; und Darmdehnung.

9. Verwendung eines β-D-Galaktosidase-Enzyms für die Herstellung eines diagnostischen Wirkstoffes für die Diagnose einer Kolikart in einem Kind, das ein oder mehrere Symptome zeigt, die charakteristisch für unspezifische Kolik sind, wobei die Diagnose folgende Schritte umfasst:
a) Messung des Wasserstoffgehalts der Atemluft in dem Kind;
b) orale Verabreichung einer flüssigen Formulierung des β-D-Galaktosidase-Enzyms separat von, simultan mit oder auf eine Laktose-enthaltene Nahrung folgend;
c) Messung des Wasserstoffgehalts der Atemluft nach periodischen Intervallen von mindestens 1 Stunde; und
d) Bestimmung des Unterschieds des Wasserstoffgehalts der Atemluft im Verhältnis zur Messung vor der Fütterung.

10. Verwendung nach Anspruch 9, wobei eine Reduktion des Wasserstoffgehalts der Atemluft in einer Größenordnung um 50 % oder mehr im Verhältnis zur Messung vor der Fütterung eine Laktose-Intoleranz-basierende Kolik anzeigt.

11. Verwendung nach Anspruch 9, wobei ein Fehlschlag, auf die Enzymforrnulierung zu reagieren, wie er durch den Wasserstoffgehalt der Atemluft gemessen wird, der im Wesentlichen unverändert ist im Verhältnis zur Messung vor der Fütterung, eine andere Kolik als eine auf Laktose-Intoleranz-basierende Kolik anzeigt.

12. Verwendung nach einem der Ansprüche 9 bis 11, wobei der Wasserstoffgehalt der Atemluft nach Zeitintervallen von 30, 60, 90 und 120 Minuten nach der Laktose-basierenden Fütterung gemessen wird.

13. Verwendung nach einem der vorhergehenden Ansprüche, wobei der Wasserstoffgehalt der Atemluft mittels eines tragbaren Wasserstoffinonitors gemessen wird.

14. Verwendung nach einem der vorhergehenden Ansprüche, wobei das Enzym eine Laktase ist, die durch die kontrollierte Fermentation von *Kluyveromyces lactis* erhalten wird.

15. Verwendung nach einem der vorhergehenden Ansprüche, wobei die Diagnose durch eine Veränderung der Schreizeit bestätigt wird.

## Revendications

1. Utilisation d'une enzyme 6-D- galactosidase pour la fabrication d'un agent de diagnostic pour diagnostiquer le type de colique chez un enfant présentant un ou plusieurs symptômes caractéristiques d'une colique non spécifiée, qui comprend les étapes de :
a) mesure optionnelle chez cet enfant d'au moins un paramètre indicatif d'une colique non spécifiée ;
b) administration orale d'une formulation liquide de ladite enzyme β -D- galactosidase séparément ou simultanément ou de façon séquentielle avec une nourriture ;
c) détermination à des intervalles de temps choisis de toutes modifications dans les symptômes du type de colique chez l'enfant.

2. Utilisation selon la revendication 1, dans laquelle, une augmentation dans lesdits symptômes d'au moins 50% ou plus indique une intolérance à base de lactose.

3. Utilisation selon la revendication 1, dans laquelle lorsque la formulation de ladite enzyme ne calme pas les symptômes de colique non spécifiée, l'enfant est placé sous diététique d'alimentation faiblement allergène.

4. Utilisation selon la revendication 3, dans laquelle une augmentation de 50% ou plus dans lesdits symptômes indique une colique à base d'allergène nécessitant le maintien d'une diététique faiblement allergène.

5. Utilisation selon la revendication 3, dans laquelle un défaut de réponse indique un besoin de recherche supplémentaire.

6. Utilisation selon l'une quelconque des revendications précédentes, dans laquelle, l'enfant est alimenté par une formulation artisanale, cette formulation étant pré-incubée par ladite enzyme.

7. Utilisation selon l'une quelconque des revendications 1 à 5, dans laquelle, lorsque ledit enfant est nourri au sein, ladite enzyme est ajoutée à une quantité adéquate de lait et est ensuite administrée à l'enfant à la fin d'une tétée.

8. Utilisation selon l'une quelconque des revendications précédentes, dans laquelle le paramètre mesuré est choisi parmi un ou plusieurs des suivants : hydrogène respiratoire, pH des selles, substances réduisant les selles et distension des intestins.

9. Utilisation d'une enzyme β-D- galactosidase pour la fabrication d'un agent de diagnostic pour diagnostiquer le type de colique chez un enfant présentant un ou plusieurs symptômes caractéristiques d'une colique non spécifiée, qui comprend les étapes de :
a) mesure de l'hydrogène respiratoire chez cet enfant ;
b) administration orale d'une formulation liquide de ladite enzyme β-D-galactosidase séparément ou simultanément ou de façon séquentielle avec une nourriture contenant du lactose ;
c) mesure d'hydrogène respiratoire à des intervalles ou pendant une période d'au moins une heure ; et
d) détermination de la différence de l'hydrogène respiratoire par rapport à ladite mesure avant l'alimentation.

10. Utilisation selon la revendication 9, dans laquelle une réduction de l'hydrogène respiratoire de l'ordre de 50% ou plus par rapport à ladite mesure effectuée avant l'alimentation indique une colique basée sur l'intolérance au lactose.

11. Utilisation selon la revendication 9, dans laquelle un défaut de réponse à ladite formulation d'enzyme telle que mesurée par l'hydrogène respiratoire qui est restée sensiblement inchangée par rapport à ladite mesure effectuée avant l'administration indique une colique autre qu'une colique basée sur l'intolérance au lactose.

12. Utilisation selon l'une quelconque des revendications 9-11, dans laquelle l'hydrogène respiratoire est mesuré à des intervalles de temps de 30, 60, 90 et 120 mn, après l'alimentation à base de lactose.

13. Utilisation selon l'une quelconque des revendications précédentes, dans laquelle l'hydrogène respiratoire est mesuré au moyen d'un contrôleur d'hydrogène portable.

14. Utilisation selon l'une quelconque des revendications précédentes, dans laquelle l'enzyme est une lactase obtenue par la fermentation contrôlée de Kluyveromyces lactis.

15. Utilisation selon l'une quelconque des revendications précédentes, dans laquelle le diagnostic est confirmé par une modification de la durée des pleurs.
